# EUROPEAN PATENT SPECIFICATION

(11) **EP 4 032 101 B1**
(45) Date of publication and mention of the grant of the patent: **24.12.2025**
(21) Application number: 20786245.9
(22) Date of filing: 16.09.2020
(51) Int. Cl.: G16H 40/20

(54) **A DEVICE AND A METHOD FOR DETERMINING A PATH OF A PATIENT IN A HOSPITAL**
VORRICHTUNG UND VERFAHREN ZUR BESTIMMUNG EINES PFADES EINES PATIENTEN IN EINEM KRANKENHAUS
DISPOSITIF ET PROCÉDÉ POUR DÉTERMINER UN CHEMIN D'UN PATIENT DANS UN HÔPITAL

(30) Priority: 16.09.2019 EP 19306113
(43) Date of publication of application: 27.07.2022
(73) Proprietor: Centre Hospitalier Universitaire de Nice, 06000 Nice (FR)
(72) Inventor: HAAS, Hervé, 06000 Nice (FR); TRAN, Antoine, 06000 Nice (FR)
(74) Representative: Germain Maureau
(86) International application number: PCT/EP2020/075917
(87) International publication number: WO 2021/053044

(56) References cited:
- JP-A- 2005 165 513
- US-A1- 2009 070 137
- US-A1- 2012 232 930

## Description

### Field of the disclosure

The present disclosure relates to a device and a method for determining a path of a patient in a hospital.

### Technical background

Every year, millions of patients with a wide range of pathological conditions are inspected at emergency departments in hospitals.

For such patients, a multitude of data is commonly collected, for example, blood pressure measurements, ECG, past patient history, a summary of symptoms, and imaging data such as computed tomography (CT), ultrasound, or magnetic resonance imaging (MRI) images. These data permit to determine a treatment strategy for patient.

Hospitals are complex organizations functioning within generally strict resource limits. Emergency planning must therefore be practical and efficient.

Data regarding patients arriving at an emergency department need to be processed as soon as possible, especially to identify patients in need of urgent medical intervention and to identify the hospital resources that will be used to care the patient.

Currently, a patient arriving at the emergency department is treated within a more or less long period of time depending on his or her medical condition. The patient is identified and sorted. Several examinations take place and a diagnosis is made leading to a decision: discharged or hospitalized.

However, this method is not optimized when several people consult at the same time or in the case of mass casualties' events which require specific treatment tools according to the severity of the condition.

Therefore, an optimization of emergency management planning and response is required in order to assist physicians.

It is known from the prior art several methods for providing a decision-making support to physicians in order to optimize emergency management planning and response.

The patent document US2017/0242973 discloses an electronic triage method called "E-triage" that determines the probability of being assigned in a category with a given probability, e.g. reanimation admission, surgical admission, hospitalization. The method includes risk assessment and assigns each of the patients a triage score.

The patent document US2018/0315182 discloses a method of assessing a patient's condition and/or predicting its medical progress. This method uses a classifier to diagnose and/or make a prognosis for one or more patients in an emergency situation. This method collects patient-specific data, and uses past patient history in order to output different kinds of information, e.g. a sorting order, query the missing data to refine the diagnosis or prognosis, data relevant to decision-making support, etc.

US 2012/232930 A1 relates to a clinical decision support system performs a similarity search to determine the probable outcome of applying on a current patient those clinical actions that were performed on similar patients.

US 2009/070137 A1 relates to a optimizing status of a care plan by defining worksteps associated by a rule-based process.

JP 2005 165513 A relates to a hospital information system efficiently managing progress information of a clinical path.

The present disclosure concerns methods and devices that enable to capitalize on experience feedbacks in order to improve the management of the hospital resources and effectively determine a path of a patient in a hospital.

### Summary of the disclosure

In what follows, the term "comprise" is synonym of (means the same as) "include" and "contains", is inclusive and open, and does not exclude other non-recited elements.

According to a first aspect, the disclosure relates to a computer-implemented method for determining a path of a patient in a hospital comprising the following steps:
- receiving administrative and/or subjective data of the patient;
- comparing said administrative and subjective data with administrative and subjective data of previously treated patient stored in a database to determine previously treated patients having a given level of similarity with the patient, wherein said database comprises, for each previously treated patient, a recorded path;
- collecting hospital resources availability data;
- determining an initial path of the patient in the hospital wherein said initial path includes calculated probabilities of using specific hospital resources in a given sequence, based on the recorded path of said previously treated patients having said level of similarity with the patient, and said hospital resources availability data.

In what follows, the term "hospital" is not necessarily limited to a single medical structure but may also include networks of medical structures that may share medical resources.

In what follows, the term "hospital resources" comprises in particular the medical staff and the medical units and/or devices; for example, the hospital resources may comprise physician, a specialist physician, a surgeon, a nurse, a subspecialist, an unlicensed assistive personnel and their associated care units which associated, for example, but not limitated to, with one of the following specialties : addiction, alcoholology, algology, allergology, anatomy and pathological cytology, andrology, anesthesiology-surgical resuscitation, angiology, biochemistry, medical biology and physiology, cardiology, surgery, digestive surgery, infant surgery, maxillofacial surgery and stomatology, orthopaedic and traumatological surgery, plastic surgery, plastic surgery reconstructive and aesthetic, thoracic and cardiovascular surgery, urological surgery, vascular surgery, visceral surgery, contactology, coronary angiography, dentistry, dermatology and venereology, endocrinology and metabolism, functional explorations, gastroenterology and hepatology, gynecology-obstetrics, hematology, infectious and tropical diseases, occupational medicine, internal medicine, forensic medicine, nuclear medicine, physical and rehabilitation medicine, neonatology, nephrology, neurosurgery, neurology, neuropsychiatry, odontology, oncology, ophthalmology, orthodontics, orthoptics, osteopathy, oto-rhinolaryngology, otology, otoneurology, periodontics, periodontology, pediatrics, pharmacy, pharmacology, phlebology, pulmonology, posturology, proctology, psychiatry, radiology, diagnosis and medical imaging radiotherapy, medical resuscitation, rheumatology, palliative care, stomatology, strabology, tobacology, urology, venereology which also comprises the availability of their associated medical devices known from the skilled man such as, for example, but not limited to, blood pressure measurement device, ECG measurement device, computed tomography device, ultrasonic measurement device, or magnetic resonance imaging device. Moreover, resource comprises mobile medical unit or mobile paramedical unit.

In what follows, the term "hospital resources availability data" refers to data describing availability of the hospital resources. At a basic level, such hospital resources availability data may consist of a binary code. At a more complex level, such hospital resources availability data may comprise a nomenclature attributing to each hospital resource an expected waiting time before the specific resource is available.

In what follows, "administrative data of a patient", or "administrative data", generally refers to demographic data such as, for example, but not limited to, age, gender, location. These administrative data may also include socio-demographic data.

In what follows, "subjective data of a patient", or "subjective data", may refer to, for a conscious patient, symptoms described by the patient or complaint(s) made by the patient. The subjective data may include also medical/surgical history (smoking, alcohol consumption, high blood pressure, drug use, current medicines being used, or others), chronic conditions and mode of arrival.

In what follows, the term "database" refers to an organized collection of data. In particular it may refer to a physical storage medium such as, but not limited to, read-write random-access memory, typically DRAM (dynamic RAM) or other forms of fast but temporary storage, or hard disk drives, optical disc drives, and other devices slower than RAM but non-volatile (retaining contents when powered down).

In what follows, the term "previously treated patient path" refers to the sequence of hospital resources actually used during the stay of such previously treated patient in the hospital.

The computer-implemented method by using a longitudinal analysis using previous patient data and cross-sectional analysis using data from patients with similar profiles permits to predict the resources that the patient would consume and to determine an initial path of the patient in the hospital.

In situations of crisis, for example when a hospital gets saturated within the frame of a pandemic, such improvement may turn out to have a large impact on the quality of a diagnosis or a therapy that is provided to a patient. One objective of the method according to the present disclosure is thus to improve the quality of a diagnosis/therapy provided to a patient, by efficiently reorganizing patient fluxes at a scale of *e.g.,* a city or a region.

Moreover, such computer-implemented method permits to improve patient triage based on resource predictions and to improve patient path in order to fluidize the path, reduce waiting time which can lead to an improvement of patient satisfaction.

The computer-implemented method further comprises during the course of the path of the patient within the hospital, the steps of:
- receiving objective data characterizing a pathological condition of the patient;
- comparing said objective data with objective data of said previously treated patients having said level of similarity with the patient to determine a subset of previously treated patients having said level of similarity with the patient;
- collecting hospital resources availability data;
- determining an initial path of the patient in the hospital wherein said initial path includes calculated probabilities of using specific hospital resources in a given sequence, based on the recorded path of said previously treated patients having said level of similarity with the patient, and said hospital resources availability data;
- recording in real time an actual path followed by the patient in the hospital;
- updating hospital resources availability data based on said actual path followed by the patient;
- updating the path) of the patient based on the recorded path of said patients within the subset and hospital resources availability data

In what follows, "objective data of a patient", or "objective data", may refer to physical quantities which may characterize a pathological state or condition of a patient such as, but not limited to, temperature, heart rate, systolic blood pressure, respiratory rate, oxygen saturation, glasgow score. The objective data of said previously treated patients having said level of similarity with the patient, and/or the recorded paths of the patients within the subset, may for example be stored in the same above-mentioned database.

According to one or more embodiments, the administrative and/or subjective data and/or objective data and/or hospital resources availability data are constituted of items having relative weights, wherein the calculated probabilities included in the initial path are calculated on the basis of said weights.

Updating hospital resources availability data may be performed in real time, and is based on the recording in real time of the actual path followed by the patient. The method further comprises the step of updating the path of the patient, based on said updated hospital resources availability data. By providing an updated path to a patient, the method may therefore be iterative and self-improving, which may be particularly advantageous, for example, in case of mass casualties' events and/or pandemic.

In one or other embodiments, the computer-implemented method further comprises the steps of:
- comparing the actual path followed by the patient in the hospital with the initial path by identifying differences between hospital resources actually used and the initially calculated probabilities of using specific hospital resources;
- recording said identified differences.

For example, according to one or more embodiments of the method wherein the administrative data and/or subjective data and/or objective data and/or hospital resources availability data are constituted of items, and wherein the calculated probabilities included in the initial path are calculated on the basis of said weights, the method may further comprise the steps of:
- based on the recorded identified differences, reassessing the weights of said items constituting the administrative and/or subjective data and/or objective data and/or hospital resources availability data;
- based on the reassessed weights, recalculating said probabilities; and
- updating the path of the patient by including therein said recalculated probabilities.

In one or other embodiments, the computer-implemented method further comprises the steps of:
- determining an emergency level of the patient;
- using said emergency level to determine said initial path of the patient.

In one or other embodiments, the step of determining an emergency level, or priority treatment level, comprises comparing objective data items used to characterize a life-threatening condition or a functional-threatening condition or any other predefined condition.

In one or other embodiments, said emergency level is sorted with respect to a scale comprising a plurality of levels, for example at least three levels, describing conditions between a life-threatening prognosis and an absence of emergency.

Determining an emergency level of the patient may be used to adapt in real-time the actual path of the patient.

In one or other embodiments, the computer-implemented method further comprises the steps of:
- calculating an expected presence time of the patient within the hospital based on the use of the hospital resources.

In one or other embodiments, said steps of comparing the administrative and subjective data of the patient with subjective and administrative data of previously treated patient and said step of determining an initial path of the patient are performed using a neural network.

In one or other embodiments, recorded differences between said initial path and an actual path of the patient within the hospital are used to train said neural network.

In one or other embodiments, said neural network is chosen in a list comprising: a recurrent neural network, a convolutional neural network, a fully convolutional neural network and their combinations thereof.

In what follows, the term "neural network" refers to a mathematical structure taking an object as input and producing another object as output though a set of linear and non-linear operations called layers. Such structures have parameters which can be tuned through a learning phase so as to produce a particular output, and are for instance used for classification purposes. The input is then the object to categorize, and the output the probabilities to pertain in each of the categories.

In what follows, the term "convolutional neural network" refers to a neural network which is partly composed of convolutional layers, i.e. layers which apply a convolution on their input. A "fully convolutional neural network" refers to a convolutional neural network in which all linear operations are convolutions. A "recurrent convolutional neural network" is a network composed of two sub-networks: a convolutional neural network which extracts features, and a neural network on top of it which accumulates during the course of time the outputs of the convolutional neural network in order to provide a refined output.

Using a neural network enables to get an output even if the input data are partial. Further, a neural network can be dynamically trained and therefore be continuously improved by recorded differences between real outputs and expected outputs.

In one or other embodiments, medical or medical related data such as administrative data, objective data, subjective data or hospital resources availability data defined above may be constituted of items.

Such items may be combined to form a mathematical object such as a vector or a matrix to be used by the neural network.

When recorded in a database, such items may be recorded according to a standardized norm which may be international, regional, or national.

Such items may be closed, i.e. there is no possible variability or interpretation from one patient to another when these items are used. The different kinds of above-mentioned data may therefore be broken down into as many items as necessary to report on the patient's condition, taking into account the evolution of medical referential and the local medical referential. The number of items is, nonetheless, limited. Examples of what these items could be have been given previously in the respective definitions of the different kinds of data.

In one or other embodiments, the level of similarity between administrative and subjective data is based on one or a plurality of predetermined criteria.

In one or other embodiments, said level of similarity is based on an overlapping of at least 50% of the items of the patient with said corresponding items of the previously treated patients, preferentially at least 70%, and even more so preferentially at least 90%.

In one or other embodiments, items are weighted according to their importance in determining the initial path of the patient in the hospital. For example, items are distributed on a scale with higher levels corresponding to higher relative weights.

In one or other embodiments, the level of similarity is based on an overlapping of at least 50% of the most weighted items of the patient with said most weighted items of the previously treated patients, preferentially at least 70%, and even more so preferentially at least 90%. The most weighted items may comprise the first 5%, first 10%, first 20%, first 30% or first 40% weighted items.

Such determination of the level of similarity is not limited to the most weighed items but may also comprise a level by level comparison. For example, determining the level of similarity may include a sub-comparison using the overlapping criteria described above.

In one or other embodiments, the method further comprises the step of determining the probability for the patient to be discharged based on administrative and/or subjective data and/or objective of the patient.

In one or other embodiments, the method further comprises the step of determining the probability for the patient to have a medical complication based on administrative and/or subjective data and/or objective of the patient.

In one or other embodiments, the method further comprises the step of determining the probability for the patient to come back to the hospital in the coming days, preferably in the next seven days, based on administrative and/or subjective data and/or objective of the patient.

In one or other embodiments, the method further comprises the step of displaying at least one of the following outputs: an initial path and/or updated paths of a patient, hospital resources expected to be used by the patient with a given probability, a priority treatment level, a probability of using specific hospital resources, an expected time within the hospital, a probability for the patient to be hospitalized or discharged, a probability for the patient to have a medical complication, a probability for the patient to come back to the hospital.

In one or other embodiments, the patient is an infant, or a child, or his age allows a pediatric care in the hospital.

According to a second aspect, the disclosure relates to data processing devices configured to perform computer-implemented methods according to the first aspect.

The data processing device comprises:
- at least one input unit for inputting said administrative and subjective data of the patient;
- said database;
- at least one display unit;
- at least one communication unit configured to:
   ∘ collect said hospital resources availability data;
   ∘ collect an actual path followed by the patient;
      update (144) the hospital resources availability data
   ∘ send at least said initial path of the patient to the at least one display unit;
- a processor configured to compare the administrative and subjective data of the patient with subjective and administrative data of previously treated patient and determine said initial path of the patient, and update (134) the path (115) based on the updated hospital resources availability data.

In what follows, the term "display unit" refers to a device for presentation of information in visual and/or tactile form.

In what follows, the term "input unit" refers to a piece of computer hardware equipment used to provide data and control signals to an information processing system such as a computer or information appliance such as, for example, but not limited to, keyboards, mouse, scanners, digital cameras, joysticks, and microphones.

In one or other embodiments, said processor comprises a neural network.

In one or other embodiments, one of said at least one input unit is remote. Said remote input unit may be hosted by a mobile medical unit or a mobile paramedical unit.

The use of one or several display units may enable to notify the medical staff within the hospital of different information such as, but not limited to, the resource currently used by the patient, the next resource to be used or to inform if the priority level has changed.

In one or other embodiments, the data processing device further comprises a clustering unit configured to cluster paths of a plurality of patients.

In one or other embodiments, the clustering unit is further configured to determine if resources can be strained by said plurality of patients.

In one or other embodiments, the database stores administrative, subjective and objective data of all previous treated patients.

In one or other embodiments, the database stores each initial path and each updated initial path, and the actual path followed.

In one or other embodiments, the database stores each diagnosis made for the patient.

In one or other embodiments, the database stores criteria for selecting specific previously treated patients.

In one or other embodiments, the communication unit is a wireless communication unit.

In one or other embodiments, the data processing device comprises a GPS unit or a GPS-like unit configured to be worn by the patient.

According to one or more embodiment, the GPS unit or GPS-like unit are configured to communicate through wireless communication with the wireless communication unit.

The GPS unit or a GPS-like unit may be used to follow the actual path followed by the patient.

According to a third aspect, the disclosure relates to a computer program for determining an initial path of a patient in a hospital comprising instructions which, when the program is executed by a data processing device according to the present disclosure, cause the data processing device to carry out the computer-implemented method according to the present disclosure.

According to a fourth aspect, the disclosure relates to a computer-readable medium for determining an initial path of a patient in a hospital comprising instructions which, when executed by a data processing device according to the present disclosure, cause the data processing device to carry out the computer-implemented method according to anyone of the present disclosure.

The embodiments described above are not exhaustive. In particular, it is understood that additional embodiments can be considered on the basis of different combinations of the explicitly described embodiments. Unless otherwise specified in the present disclosure, it will be apparent to the skilled person that all the embodiments described above can be combined together.

### Brief description of the drawings

The present disclosure will be better understood and other advantages and embodiments will become clear on reading the description that follows, given purely by way of indication and in no way limiting, and by referring to the appended FIGs. in which:
- FIG. 1 is a device according to one or more embodiments of the present disclosure.
- FIG. 2 is a schematic representation of a network of hospital structures.
- FIG. 3 is an example of a structure for a basic neural network.
- FIG. 4 is a schematic representation of patient flow in a hospital according to one or more embodiments of the present disclosure.

### Detailed description of embodiments

Embodiments of the present disclosure will now be described in detail with reference to the accompanying FIGs. In the following detailed description of embodiments of the present disclosure, numerous specific details are set forth in order to provide a more thorough understanding of the present disclosure. However, it will be apparent to one of ordinary skill in the art that the present disclosure may be practiced without these specific details. In other instances, well-known features have not been described in detail to avoid unnecessarily complicating the description.

The following description provides a particular, non-limiting example of a method and a device for determining a path of a patient in a hospital.

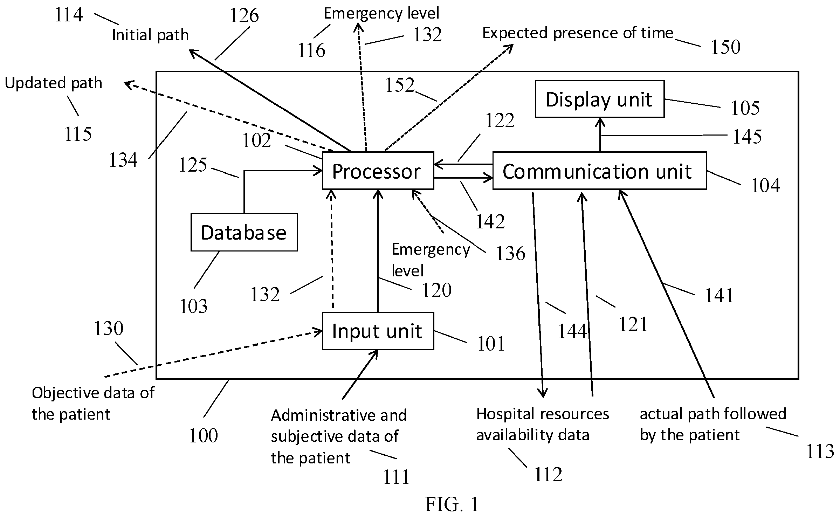

FIG. 1 shows a data processing device 100 according to one or more embodiments of the present disclosure.

Said device 100 comprises at least one input unit 101 for receiving administrative and subjective data 111 of a patient.

The device 100 further comprises a processor 102, a database 103 that records administrative and subjective data of previously treated patient, at least one display unit 105 and a communication unit 104.

The communication unit may refer as a single component but may comprise a plurality of distinct electronic components. The communication unit can for instance be, or comprise, a processing unit such as a central processing unit (CPU), a graphic processing unit (GPU), a Field Programmable Gate Array (FPGA), an Application-Specific Integrated Circuit (ASIC) and/or any type of such processing unit known in the art. The communication unit and other elements of the device may be connected together, directly or through interconnection electronic components.

The communication unit is configured to collect (step 121) hospital resources availability data 112, collect (step 141) an actual path 113 followed by the patient and send (step 145) an initial path 114 of the patient to the at least one display unit 105.

The processor 102 may comprise a neural network. The processor 102 is configured to compare (step 125) the administrative and subjective data 111 of the patient with subjective and administrative data of previously treated patient and determine said initial path of the patient. The processor 102 is also configured to determine (step 126) an initial path 114 of the patient in the hospital wherein said initial path includes calculated probabilities of using specific hospital resources in a given sequence, based on the recorded path of said previously treated patients having a level of similarity with the patient, and said hospital resources availability data.

In other embodiments, the device 100 is configured to receive (step 132) objective data 130 characterizing a pathological condition of the patient. The processor 102 is therefore configured to compare (step 125) said objective data with objective data of said previously treated patients having said level of similarity with the patient to determine a subset of previously treated patients and is configured to update (step 134) the path 115 of the patient based on the recorded path of said patients within the subset, and hospital resources availability data.

The device 100 is configured to record in real time an actual path followed by the patient in the hospital and the processor 102 is configured to update (step 144) hospital resources availability data based on said actual path 113 followed by the patient.

In other embodiments, the processor 102 is configured to determine (step 132) an emergency level 116 of the patient and to use (step 136) said emergency level 116 to determine said initial path 114 of the patient.

In other embodiments, the processor 102 is configured to calculate (step 152) an expected presence time 150 of the patient within the hospital based on the use of the hospital resources 112.

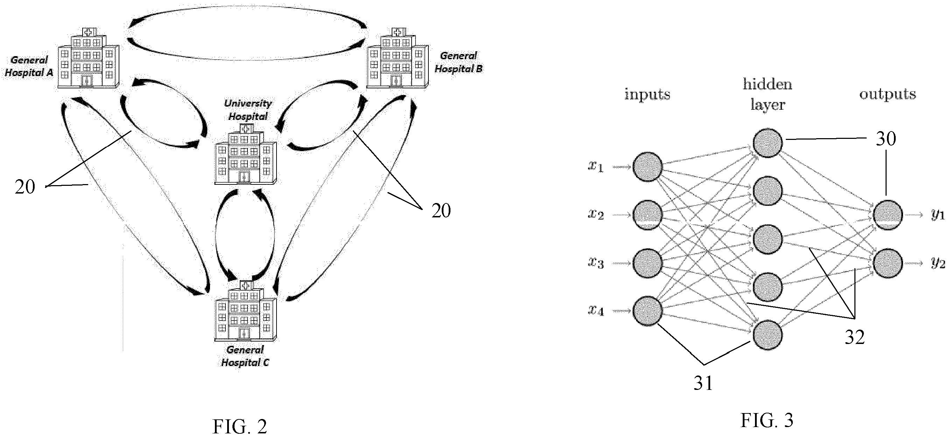

FIG. 2 shows a schematic representation of a network of medical structures. As a matter of fact, a hospital does not necessarily have all the required medical resources. This is particularly true for so called "forward hospital" or "general hospital" A, B or C which can offer only reduced hospital resources. A university hospital includes, theoretically, all necessary resources. Therefore, as shown in FIG. 2, a patient transfer 20 may occur between medical structures of a hospital in order to provide access to a particular resource to a patient.

FIG. 3 shows a neural network in a graphic form. A "neural network" is a mathematical structure taking an object as input (x1, x2, x3, x4) and producing another object as output (y1, y2) through a set of linear and non-linear operations called layers 31 which are constituted by a plurality of nodes 31.

Such structures have parameters which can be tuned through a learning phase so as to produce a particular output, and are for instance used for classification purposes. The input is then the object to categorize, and the output the probabilities to pertain in each of the categories.

A "convolutional neural network" is a neural network which is partly composed of convolutional layers, i.e. layers which apply a convolution on their input.

A "fully convolutional neural network" is a convolutional neural network in which all linear operations are convolutions.

The framework used is the one of supervised learning. The aim of supervised learning is to predict an output vector Y from an input vector X. In the Applicant embodiment, X is data from a patient (a multivariate signal) as a matrix of size m x n. As for Y, in the Applicant embodiment, it can be:
- the delineation, providing a score for each sample of X to be part of one of the different waves as a matrix of size p x n;
- the scores for each label as a vector of size q;
- the set composed of both the delineation and the vector of scores.

The problem of supervised learning can also be stated as follows: designing a function f such that for any input X, f(X) ≈ Y. To this end, the function f is parametrized, and these parameters are "learned" (parameters are optimized with regards to an objective loss function, for example, by means of a gradient descent (Bishop. Pattern Recognition and Machine Learning. Springer. 2006. ISBN- 10: 0-387-3 1073-8).

A neural network is a particular type of function f, aiming at mimicking the way biological neurons work. One of the most basic and earliest neural network is the perceptron (Rosenblatt. Psychological Review . Vol. 65. No. 6. 1958. pp 386-408 ). From the input X, it computes linear combinations (i.e. weighted sums) of the elements of X through a multiplication with a matrix W, adds an offset B, and then applies a non-linear function σ, such as for instance a sigmoid, on every element of the output: f(X) = σ(WX + B).

The parameters which are learned in a perceptron are both W and B. In practice, more general neural networks are just compositions of perceptrons: f(X) = σn(Wn... σn(W1X + B1) + Bn).

The output of a perceptron can be sent as input to another one. The input, the final output, and the intermediate states are called layers. The intermediate ones are more specifically called hidden layers, since only the input and the final output are observed. For instance, a neural network with one hidden layer can be written as: f(X) = σ2(W2σ1(W1X + B1) + B2).

As shown on FIG. 3, the vector X (x₁, x₂, x₃, x₄) enters the network as the input layer, each element of the hidden layer is then computed from linear combinations of all elements of X (hence all the links 32), and the element of the output layer are then computed from linear combinations of all elements of the hidden layer.

It has been shown that neural networks in their general form are able to approximate all kinds of functions (Cybenko. Math. Control Signals Systems. Vol. 2, 1989. pp 303-314). The term "deep learning" is used when a neural network is composed of many layers (although the threshold is not perfectly defined, it can be set to about ten). This field arose mostly in the last decade, thanks to recent advances in algorithms and in computation power.

Convolutional neural networks are a particular type of neural networks, where one or more of the matrices Wi which are learned do not encode a full linear combination of the input elements, but the same local linear combination at all the elements of a structured signal through a convolution (Fukushima, Biol. Cybernetics, Vol. 36, 1980, pp 193-202, LeCun et al., Neural Computation, Vol. 1, 1989, pp 541-551). Most convolutional neural networks implement a few convolutional layers and then standard layers so as to provide a classification. A network which only contains convolutional networks is called a fully convolutional neural network.

Finally, a recurrent convolutional neural network is a network composed of two sub-networks: a convolutional neural network which extracts features, and a neural network on top of it which accumulates through time the outputs of the convolutional neural network in order to provide a refined output.

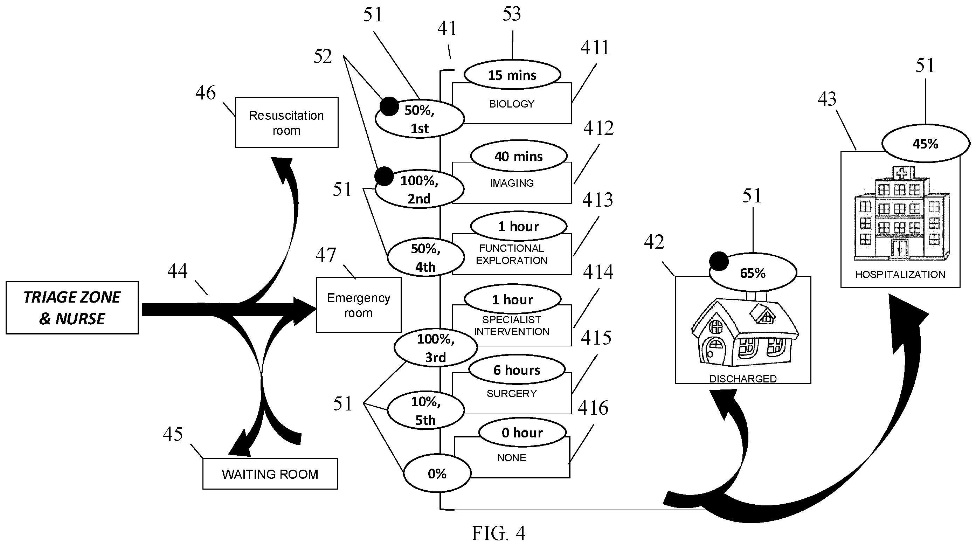

FIG. 4 represents a patient flow in a hospital according to an example.

In this example, hospital resources include medical units 41: a biology unit 411, an imaging unit 412, a functional exploration unit 413, a surgery unit 415.

The hospital resources also include specialists 414 associated to one with the above cited medical units.

416 shows a diagnosis made without further examination.

When a patient arrives at an emergency department of the hospital, a triage is done based on subjective data. The patient is received by a triaging nurse when administrative and subjective data from the patient are requested. Such data and associated items are obtained through a standardized interview in order to file items in a standardized document. For example, a chest pain or an infant is the subject of sustained attention by an emergency service.

The completion of the items in the standardized document, which can be in the form of a digital questionnaire displayed on a display unit is critical. Indeed, this document will accompany the patient until at least the use of specific resources that will allow the items in this document to be updated. The number of items to be completed may vary from one hospital to another and even from one country to another. It depends on the evolution of the procedure, standards and medical knowledge.

According to the estimated emergency, a patient may be oriented to a waiting room 45, to an emergency room 47, or to a resuscitation room 46 for life-threatening conditions. In these two first cases, the patient moves back and forth between the waiting or the emergency room and the resources that the emergency service considers that should be used in order to make a diagnosis. After a diagnosis, the emergency department discharges the patient 42 who can go back to his house or decides to admit to the hospital the patient 43. The flow of the patient in the hospital is materialized by the arrows 44.

In this particular example, a patient with right wrist pain and redness arrived at the reception desk of a hospital emergency department.

First, administrative and/or subjective data 111 of the patient are entered into the system to allow the method to be implemented 120.

Based on the data entered into the system, a first vector defining the patient's medical condition is created. The vector comprises the following elements: pain in the right wrist, redness.

The vector is used as an input in a neural network in order to determine by comparing 125 previously treated patient having, stored in a database 103, a given level of similarity with the patient, wherein for each previously treated patient a previously treated patient path is recorded.

As an output, a second vector comprising previously treated patient path wherein said previously treated patient have a given level of similarity with the patient is generated.

A third vector defining the availability of the hospital resources is created after hospital resources availability data 112 collection. This vector comprises an actual waiting time before a specific resource becomes available associated with each of the resources 411, 412, 413, 414, 415, 416 of the hospitals.

In the described embodiment, the expected waiting time is mentioned inside ellipses 53.

The second and third vectors are used as inputs in the neural in order to determine (step 126) an initial path 114 of the patient in the hospital wherein said initial path includes calculated probabilities of using specific hospital resources in a given sequence, based on the recorded path of said previously treated patients having said level of similarity with the patient, and said hospital resources availability data.

Based on the vectors, the neural network outputs an initial path 114 comprising the probabilities to use a specific resource and a given sequence to use specific hospital resources.

In this example, the probability to use an imaging resource such as IRM is 100% whereas the IRM is not available before 1 hour. The probability to use a biology resource such as a blood test is 50% whereas the biology resource is not available before 15 minutes.

Therefore, the output 51 of the method is the initial path 114:
- resource "biology" : 50 %, 1^{st} stage
- resource "imaging" : 100 %, 2^{nd} stage
- resource "specialist intervention" : 100 %, 3^{rd} stage
- resource "functional exploration" : 50 %, 4^{th} stage
- resource "surgery" : 10 %, 5^{th} stage
- resource "none" : 0%

However, the recorded actual path 113 represented by element 52 shows that only "imaging" resource and "biology" resource were used prior to discharge the patient.

Such recorded differences can be used to train the neural network through a supervised learning in order to converge to the optimized initial path as closely as possible to the actual path.

## Claims

1. A computer-implemented method for determining a path of a patient in a hospital, the method comprising the following steps:
- receiving (120) administrative and/or subjective data (111) of the patient;
- comparing (125) said administrative and subjective data (111) with administrative and subjective data of previously treated patient stored in a database (103) to determine previously treated patients having a given level of similarity with the patient, wherein said database (103) comprises, for each previously treated patient, a recorded path;
- collecting (122) hospital resources availability data (112);
- determining (126) an initial path (114) of the patient in the hospital wherein said initial path includes calculated probabilities of using specific hospital resources in a given sequence, based on the recorded path of said previously treated patients having said level of similarity with the patient, and said hospital resources availability data (112);
- recording in real time an actual path (113) followed by the patient in the hospital;
- updating (144) hospital resources availability data (112) based on said actual path (113) followed by the patient;
- based on the updated hospital resources availability data (112), updating (134) the path (115).

2. The computer-implemented method according to claim 1, further comprising, during the course of the path of the patient within the hospital, the steps of:
- receiving (132) objective data (130) characterizing a pathological condition of the patient;
- comparing (125) said objective data with objective data of said previously treated patients having said level of similarity with the patient to determine a subset of previously treated patients having said level of similarity with the patient;
- updating (134) the path (115) of the patient based on the recorded path of said patients within the subset and hospital resources availability data (112).

3. The computer-implemented method according to claim 1 or claim 2,
wherein the administrative and/or subjective data (111) and/or objective data (130) and/or hospital resources availability data (112) are constituted of items having relative weights, wherein the calculated probabilities included in the initial path (114) are calculated on the basis of said weights, and wherein the method further comprises the steps of:
- comparing the actual path (113) followed by the patient in the hospital with the initial path (114) by identifying differences between hospital resources actually used and the initially calculated probabilities of using specific hospital resources;
- recording said identified differences;
- based on the recorded differences, reassessing the weights of said items constituting the administrative and/or subjective data (111) and/or objective data (130) and/or hospital resources availability data (112);
- based on the reassessed weights, recalculating said probabilities; and
- updating the path (115) of the patient by including therein said recalculated probabilities.

4. The computer-implemented method according to any one of the preceding claims, further comprising the step of:
- determining (132) an emergency level (116) of the patient;
- using (136) said emergency level (116) to determine said initial path (114) of the patient.

5. The computer-implemented method according to any one of the preceding claims, further comprising the step of:
- calculating (152) an expected presence time (150) of the patient within the hospital based on the use of the hospital resources (112).

6. The computer-implemented method according to any one of the preceding claims, wherein said steps of comparing the administrative and subjective data (111) of the patient with subjective and administrative data of previously treated patient and said step of determining (126) an initial path (114) of the patient are performed using a neural network.

7. The computer-implemented method according to claim 6, wherein recorded differences between said initial path (114) and an actual path (113) of the patient within the hospital are used to train said neural network.

8. The computer-implemented method according to claim 7, wherein said neural network is chosen in a list comprising: a recurrent neural network, a convolutional neural network, a fully convolutional neural network and combinations thereof.

9. A data processing device (100) configured to perform the computer-implemented method for determining a path of a patient in a hospital according to any one of the preceding claims, comprising:
- at least one input unit (101) for inputting said administrative and subjective data (111) of the patient;
- said database (103);
- at least one display unit (105);
- at least one communication unit (104) configured to:
∘ collect (121) hospital resources availability data (112);
∘ collect (141) an actual path followed by the patient (113);
∘ update (144) the hospital resources availability data (112);
∘ send (145) at least said initial path (114) of the patient to the at least one display unit (105);
- a processor (102) configured to compare the administrative and subjective data (111) of the patient with subjective and administrative data of previously treated patient, determine said initial path (114) of the patient, and update (134) the path (115) based on the updated hospital resources availability data (112).

10. The data processing device (100) according to claim 9 wherein said processor (102) comprises a neural network.

11. The data processing device (100) according to claim 9 or claim10, wherein one of said at least one input unit is hosted by a mobile medical unit or a mobile paramedical unit.

12. A computer program for determining a path of a patient in a hospital comprising instructions which, when the program is executed by a data processing device according to any one of claims 9-11, cause the data processing device to carry out the computer-implemented method according to any one of claims 1-8.

13. A computer-readable medium for determining a path of a patient in a hospital comprising instructions which, when executed by a data processing device according to any one of claims 9-11, cause the data processing device to carry out the computer-implemented method according to any one of claims 1-8.

## Patentansprüche

1. Ein computerimplementiertes Verfahren zum Bestimmen des Pfads eines Patienten in einem Krankenhaus, wobei das Verfahren die folgenden Schritte umfasst:
- Empfangen (120) administrativer und/oder subjektiver Daten (111) des Patienten;
- Vergleichen (125) der administrativen und subjektiven Daten mit administrativen und subjektiven Daten zuvor behandelter Patienten, die in einer Datenbank (103) gespeichert sind, um zuvor behandelte Patienten zu bestimmen, die einen bestimmten Grad an Ähnlichkeit mit dem Patienten aufweisen, wobei die Datenbank (103) für jeden zuvor behandelten Patienten einen aufgezeichneten Pfad umfasst;
- Sammeln (122) von Daten zur Verfügbarkeit von Krankenhausressourcen (112);
- Bestimmen (126) eines anfänglichen Pfads (114) des Patienten im Krankenhaus, wobei der anfängliche Pfad berechnete Wahrscheinlichkeiten für die Verwendung bestimmter Krankenhausressourcen in einer bestimmten Reihenfolge enthält, basierend auf dem aufgezeichneten Pfad der zuvor behandelten Patienten mit dem genannten Grad an Ähnlichkeit mit dem Patienten und den Daten zur Verfügbarkeit von Krankenhausressourcen (112);
- Aufzeichnen eines tatsächlichen Pfads (113), den der Patient im Krankenhaus zurücklegt, in Echtzeit;
- Aktualisieren (144) der Daten zur Verfügbarkeit von Krankenhausressourcen (112) basierend auf dem tatsächlichen Pfads (113), den der Patient im Krankenhaus zurücklegt, in Echtzeit;
- Aktualisieren (134) des Pfads (115) basierend auf den aktualisierten Daten zur Verfügbarkeit von Krankenhausressourcen (112).

2. Das computerimplementierte Verfahren nach Anspruch 1, das während des Behandlungsverlaufs des Patienten im Krankenhaus außerdem die folgenden Schritte umfasst:
- Empfangen (132) objektiver Daten (130), die einen pathologischen Zustand des Patienten charakterisieren,
- Vergleichen (125) der objektiven Daten mit objektiven Daten der zuvor behandelten Patienten, die den genannten Grad an Ähnlichkeit mit dem Patienten aufweisen, um eine Teilmenge der zuvor behandelten Patienten zu bestimmen, die den genannten Grad an Ähnlichkeit mit dem Patienten aufweisen,
- Aktualisieren (134) des Pfads (115) des Patienten auf der Grundlage des aufgezeichneten Pfads der Patienten innerhalb der Teilmenge und der Daten zur Verfügbarkeit von Krankenhausressourcen (112).

3. Das computerimplementierte Verfahren nach Anspruch 3, wobei die administrativen und/oder subjektiven Daten (111) und/oder die objektiven Daten (130) und/oder die Daten zur Verfügbarkeit von Krankenhausressourcen (112) aus Elementen mit relativen Gewichten bestehen, wobei die berechneten Wahrscheinlichkeiten, die in den anfänglichen Pfads (114) einbezogen werden, auf der Grundlage dieser Gewichte berechnet werden, und wobei das Verfahren ferner die folgenden Schritte umfasst:
- Vergleichen des tatsächlichen Pfads (113), den der Patient im Krankenhaus zurückgelegt hat, mit den anfänglichen Pfads (114) durch Identifizieren von Unterschieden zwischen den tatsächlich verwendeten Krankenhausressourcen und den ursprünglich berechneten Wahrscheinlichkeiten der Verwendung bestimmter Krankenhausressourcen;
- Aufzeichnung der festgestellten Unterschiede ;
- basierend auf den aufzeichnen Unterschieden die Gewichtung der Elemente der administrativen und/oder subjektiven Daten (111) und/oder der objektiven Daten (130) und/oder der Daten zur Krankenhausressourcenverfügbarkeit (112) neu bewerten;
- basierend auf den neu bewerteten Gewichtungen die Wahrscheinlichkeiten neu berechnen; und
- des Pfads (115) des Patienten unter Berücksichtigung der neu berechneten Wahrscheinlichkeiten aktualisieren.

4. Das computerimplementierte Verfahren nach einem der vorhergehenden Ansprüche, das weiterhin den folgenden Schritt umfasst:
- Bestimmen (132) einer Notfallstufe (116) des Patienten;
- Verwenden (136) des Notfallniveaus (116), um den anfänglichen Pfads (114) des Patienten zu bestimmen.

5. Das computerimplementierte Verfahren nach einem der vorhergehenden Ansprüche, das ferner den folgenden Schritt umfasst:
- Berechnen (152) einer erwarteten Anwesenheitszeit (150) des Patienten im Krankenhaus basierend auf der Nutzung der Krankenhausressourcen (112).

6. Das computerimplementierte Verfahren nach einem der vorhergehenden Ansprüche, wobei die Schritte des Vergleichens der administrativen und subjektiven Daten des Patienten mit subjektiven und administrativen Daten zuvor behandelter Patienten und der Schritt des Bestimmens (126) eines anfänglichen Pfads (114) des Patienten unter Verwendung eines neuronalen Netzwerks durchgeführt werden.

7. Das computerimplementierte Verfahren nach Anspruch 6, wobei die aufgezeichnete Unterschiede zwischen dem ursprünglichen Pfads (114) und einem tatsächlichen Pfads (113) des Patienten innerhalb des Krankenhauses verwendet werden, um das neuronale Netzwerk zu trainieren.

8. Das computerimplementierte Verfahren nach Anspruch 7, wobei das neuronale Netzwerk aus einer Liste ausgewählt wird, die Folgendes umfasst: ein rekurrentes neuronales Netzwerk, ein faltendes neuronales Netzwerk, ein vollständig faltendes neuronales Netzwerk und deren Kombinationen.

9. Eine Datenverarbeitungsvorrichtung (100), die dazu konfiguriert ist, das computerimplementierte Verfahren zum Bestimmen eines Pfads eines Patienten in einem Krankenhaus gemäß einem der vorhergehenden Ansprüche auszuführen, umfassend:
- mindestens eine Eingabeeinheit (101) zum Eingeben der administrativen und subjektiven Daten (111) des Patienten;
- die Datenbank (103);
- mindestens eine Anzeigeeinheit (105);
- mindestens eine Kommunikationseinheit (104), die dazu konfiguriert ist:
o Daten zur Verfügbarkeit von Krankenhausressourcen (112) zu sammeln (121);
o einen tatsächlichen Pfad (113) zu sammeln (141), dem der Patient gefolgt ist;
o Aktualisierung (144) Daten zur Verfügbarkeit von Krankenhausressourcen (112);
o mindestens den anfänglichen Pfad (114) des Patienten an die mindestens eine Anzeigeeinheit (105) zu senden (145);
- einen Prozessor (102), der so konfiguriert ist, dass er die administrativen und subjektiven Daten (111) des Patienten mit subjektiven und administrativen Daten zuvor behandelter Patienten vergleicht, den anfänglichen Pfad (114) des Patienten bestimmt und den Pfad (115) aktualisiert basierend auf den aktualisierten Daten zur Verfügbarkeit von Krankenhausressourcen (112).

10. Das Datenverarbeitungsgerät (100) nach Anspruch 9, wobei der Prozessor (102) ein neuronales Netzwerk umfasst.

11. Das Datenverarbeitungsgerät (100) nach Anspruch 9 oder 10, wobei eine der mindestens einen Eingabeeinheiten von einer mobilen medizinischen Einheit oder einer entfernten mobilen paramedizinischen Einheit gehostet ist.

12. Ein Computerprogramm zum Bestimmen des Pfads eines Patienten in einem Krankenhaus, das Anweisungen umfasst, die, wenn das Programm von einem Datenverarbeitungsgerät gemäß einem der Ansprüche 9-11 ausgeführt wird, das Datenverarbeitungsgerät veranlassen, das computerimplementierte Verfahren nach einem der Ansprüche 1-8 auszuführen.

13. Ein computerlesbares Medium zum Bestimmen des Pfads eines Patienten in einem Krankenhaus, das Anweisungen umfasst, die, wenn sie von einem Datenverarbeitungsgerät gemäß einem der Ansprüche 9-11 ausgeführt werden, dazu führen, dass das Datenverarbeitungsgerät das computerimplementierte Verfahren gemäß einem der Ansprüche 1-8 ausführt.

## Revendications

1. Procédé mis en œuvre par ordinateur pour déterminer un parcours d'un patient dans un hôpital, le procédé comprenant les étapes suivantes :
- réception (120) de données administratives et/ou subjectives (111) du patient ;
- comparaison (125) desdites données administratives et subjectives (111) avec des données administratives et subjectives d'un patient précédemment traité stockées dans une base de données (103) pour déterminer les patients précédemment traités ayant un niveau donné de similarité avec le patient, ladite base de données (103) comprenant, pour chaque patient précédemment traité, un parcours enregistré ;
- collecte (122) de données de disponibilité des ressources hospitalières (112) ;
- détermination (126) d'un parcours initial (114) du patient dans l'hôpital, ledit parcours initial comprenant des probabilités calculées d'utilisation de ressources hospitalières spécifiques dans une séquence donnée, sur la base du parcours enregistré desdits patients précédemment traités ayant ledit niveau de similarité avec le patient, et desdites données de disponibilité des ressources hospitalières (112) ;
- enregistrer en temps réel un parcours réel (113) suivi par le patient à l'hôpital ;
- mise à jour (144) des données de disponibilité des ressources hospitalières (112) en fonction dudit parcours réel (113) suivi par le patient ;
- mise à jour (134) du parcours (115) en fonction des données de disponibilité des ressources hospitalières (112) mises à jour.

2. Procédé mis en œuvre par ordinateur selon la revendication 1, comprenant en outre, au cours du parcours du patient au sein de l'hôpital, les étapes consistant à :
- recevoir (132) des données objectives (130) caractérisant un état pathologique du patient ;
- comparer (125) lesdites données objectives avec des données objectives desdits patients précédemment traités ayant ledit niveau de similarité avec le patient pour déterminer un sous-ensemble de patients précédemment traités ayant ledit niveau de similarité avec le patient ;
- mise à jour (134) du parcours (115) du patient en fonction du parcours enregistré desdits patients au sein du sous-ensemble et des données de disponibilité des ressources hospitalières (112).

3. Procédé mis en œuvre par ordinateur selon la revendication 1 ou 2,
dans lequel les données administratives et/ou subjectives (111) et/ou les données objectives (130) et/ou les données de disponibilité des ressources hospitalières (112) sont constitués d'éléments ayant des poids relatifs, dans lequel les probabilités calculées incluses dans le parcours initial (114) sont calculées sur la base desdits poids, et dans lequel le procédé comprend en outre les étapes consistant à :
- comparer le parcours réel (113) suivi par le patient à l'hôpital avec le parcours initial (114) en identifiant des différences entre les ressources hospitalières réellement utilisées et les probabilités initialement calculées d'utiliser des ressources hospitalières spécifiques ;
- enregistrer les différences identifiées ;
- sur la base des différences enregistrées, réévaluer les poids desdits éléments constituant les données administratives et/ou subjectives (111) et/ou les données objectives (130) et/ou les données de disponibilité des ressources hospitalières (112) ;
- sur la base des poids réévalués, recalculer lesdites probabilités ; et
- mettre à jour le parcours (115) du patient en y incluant lesdites probabilités recalculées.

4. Procédé mis en œuvre par ordinateur selon l'une quelconque des revendications précédentes, comprenant en outre l'étape consistant à :
- déterminer (132) un niveau d'urgence (116) du patient ;
- utiliser (136) ledit niveau d'urgence (116) pour déterminer ledit parcours initial (114) du patient.

5. Procédé mis en œuvre par ordinateur selon l'une quelconque des revendications précédentes, comprenant en outre l'étape consistant à :
- calculer (152) un temps de présence attendu (150) du patient au sein de l'hôpital sur la base de l'utilisation des ressources hospitalières (112).

6. Procédé mis en œuvre par ordinateur selon l'une quelconque des revendications précédentes, dans lequel lesdites étapes de comparaison des données administratives et subjectives (111) du patient avec les données subjectives et administratives du patient précédemment traité et ladite étape de détermination (126) d'un parcours initial (114) du patient sont réalisées à l'aide d'un réseau neuronal.

7. Procédé mis en œuvre par ordinateur selon la revendication 6, dans lequel les différences enregistrées entre ledit parcours initial (114) et un parcours réel (113) du patient au sein de l'hôpital sont utilisées pour entraîner ledit réseau neuronal.

8. Procédé mis en œuvre par ordinateur selon la revendication 7, dans lequel ledit réseau neuronal est choisi dans une liste comprenant : un réseau neuronal récurrent, un réseau neuronal convolutif, un réseau neuronal entièrement convolutif et leurs combinaisons.

9. Dispositif de traitement de données (100) configuré pour mettre en œuvre le procédé mis en œuvre par ordinateur pour déterminer un parcours d'un patient dans un hôpital selon l'une quelconque des revendications précédentes, comprenant :
- au moins une unité d'entrée (101) pour entrer lesdites données administratives et subjectives (111) du patient ;
- ladite base de données (103) ;
- au moins une unité d'affichage (105) ;
- au moins une unité de communication (104) configurée pour :
o collecter (121) des données de disponibilité des ressources hospitalières (112) ;
o collecter (141) un parcours réel (113) suivi par le patient ;
o mettre à jour (144) les données de disponibilité des ressources hospitalières (112) ;
o envoyer (145) au moins ledit parcours initial (114) du patient à l'au moins une unité d'affichage (105) ;
- un processeur (102) configuré pour comparer les données administratives et subjectives (111) du patient avec les données subjectives et administratives du patient précédemment traité, déterminer ledit parcours initial (114) du patient et mettre à jour (134) le parcours (115) en fonction des données de disponibilité des ressources hospitalières (112) mises à jour.

10. Dispositif de traitement de données (100) selon la revendication 9, dans lequel ledit processeur (102) comprend un réseau neuronal.

11. Dispositif de traitement de données (100) selon la revendication 9 ou 10, dans lequel une de l'au moins une unité d'entrée est hébergée par une unité médicale mobile ou une unité paramédicale mobile.

12. Programme informatique permettant de déterminer un parcours d'un patient dans un hôpital, comprenant des instructions qui, lorsque le programme est exécuté par un dispositif de traitement de données selon l'une quelconque des revendications 9 à 11, amènent le dispositif de traitement de données à exécuter le procédé mis en œuvre par ordinateur selon l'une quelconque des revendications 1 à 8.

13. Support lisible par ordinateur pour déterminer un parcours d'un patient dans un hôpital comprenant des instructions qui, lorsqu'elles sont exécutées par un dispositif de traitement de données selon l'une quelconque des revendications 9 à 11, amènent le dispositif de traitement de données à exécuter le procédé mis en œuvre par ordinateur selon l'une quelconque des revendications 1 à 8.
